# EUROPEAN PATENT SPECIFICATION

(11) **EP 1 737 439 B1**
(45) Date of publication and mention of the grant of the patent: **24.08.2011**
(21) Application number: 05731707.5
(22) Date of filing: 31.03.2005
(51) Int. Cl.: A61K 31/19, A61K 31/191, A61K 31/513, A61K 33/06, A61P 17/00, A61P 17/06, A61P 17/08

(54) **CALCIUM SALTS FOR THE TREATMENT OF PSORIASIS, DERMATITIS AND DANDRUFF**
CALCIUMSALZE ZUR BEHANDLUNG VON PSORIASIS, DERMATITIS UND SCHUPPEN
SELS DE CALCIUM POUR LE TRAITEMENT DU PSORIASIS, DES DERMATITES ET DES PELLICULES

(30) Priority: 02.04.2004 IT MI20040665
(43) Date of publication of application: 03.01.2007
(73) Proprietor: POLICHEM S.A., 1526 Luxembourg (LU)
(72) Inventor: GOBBI, Maria, Rosa, I-22073 Fino Mornasco (IT)
(74) Representative: Minoja, Fabrizio
(86) International application number: PCT/EP2005/003402
(87) International publication number: WO 2005/097084

(56) References cited:
- WO-A-96/19228
- WO-A-03/006031
- FR-A- 2 726 187
- MICHAEL PARANZINO: 'A New Direction For Psoriasis research ?', [Online] Retrieved from the Internet: <URL:www.medicalnewstoday.com/articles/5143 .php> [retrieved on 2008-11-06]

## Description

The present invention relates to the use of calcium trifluoroacetate, for the preparation of a topical medicament for the treatment of psoriasis, erythematous dermatitis, seborrheic dermatitis, dandruff.

Psoriasis is a chronic disease of the skin affecting about 2% of population in industrialized countries. It usually appears in the form of erythematous plaques coated by typical silvery scales. The lesions of the skin are characteristically located on the scalp, the joints, the hands and feet, although other body areas can be affected.

Among the various forms of psoriasis, particularly invalidating are the erythrodermal form which is characterized by pyrexia, strong inflammation and scaling, and tends to affect very large body areas, and the form known a psoriatic arthritis which is associated with articular pain and, in some cases, osteolysis and ankylosis. Psoriasis can sometimes induce lesions of the nail, the so called psoriatic onychopathy.

Onset of psoriasis can occur at any age, and has the same incidence in both sexes.

To date, no therapeutical treatments of proven effectiveness are available: among these, topical administration of salicylic acid or other keratolytics, vitamin D3, eosin, tar ointments, PUVA (psoralens and high-intensity ultraviolet) therapy, Tazarotene. Retinoic acid or derivatives thereof such as isotretinoin, acitretinoin and the like are used systemically.

Therefore, there still is the need for more effective treatments having less side effects, also considering that to date no resolvent treatments are available to the complex therapeutical problem of psoriasis, whose etiology is still unknown and in which self-immune, genetic and hyperproliferative components are involved.

Seborrheic dermatitis usually appears in the form of dry or greasy dandruff (scaling of the scalp); severe cases involve scaling along the hairline, ears, eyebrows, nasolabial folds. Treatment usually comprises the use of sulfur, selenium or zinc compounds, corticosteroids and ketoconazole.

It has now been found that long-lasting therapeutical results can be attained in short times and with no remarkable side effects by topical administration of calcium trifluoroacetate on psoriatic lesions, erythematous and seborrheic dermatitis lesions and scaling areas of the scalp, particularly on areas from which the squamous tissues have previously been removed.

Calcium trifluoroacetate was recently suggested as an antitumor drug for the parenteral administration (WO 03/00603 1) calcium chloride has been used in the topical treatment of psariasis (FK2726187)

The calcium salt will be applied to the skin suitably formulated according to conventional techniques, for example in the form of ointments, creams, lotions, powders, gel, spray, foams, occlusive patches, shampoos and the like. If desired, calcium trifluoroacetate will be adsorbed on plasters, bandages or medicated tissues or formulated with film-forming and bioadhesive agents.

The concentrations of the salt in the formulations may range within wide limits, generally from 0.1 to 20% by weight, preferably from 1 to 10% by weight.

The topical administration may be carried out once or twice a day for times ranging from one week to two-three months in the most serious cases.

The following examples illustrate the invention in greater detail.

### EXAMPLE 1

Cream-base formulations were prepared, containing different concentrations of calcium trifluoroacetate, in particular 1%, 2.5% and 5% concentrations.

Said creams were tested on 3 healthy volunteers, which repeatedly distributed said creams on healthy skin for various consecutive days. As no alterations or disturbs were observed, treatment was carried out on subjects suffering from cutaneous psoriasis.

The treatment was effected on 5 subjects, with various size lesions and in which the disease was recurrent. The treatment with the cream containing calcium trifluoroacetate was carried out on the skin previously subjected to keratolytic treatments to remove the corneous layer and expose a delicate skin, which showed redness due to the angiogenesis typical of this disease.

Treatment was effected distributing a thin layer of cream on the affected area, twice a day, in particular using the preparation containing 2.5% of calcium trifluoroacetate.

Days and weeks after the treatment, a reduction in redness and hyperkeratosis was observed, thereby proving the effectiveness of calcium trifluoroacetate in preventing recurrence of psoriasis.

### EXAMPLE 2

Shampoo formulations were prepared, containing different concentrations of calcium trifluoroacetate, in particular 5%, 10% and 15% concentrations.

Said shampoos were tested on 3 healthy volunteers, which used them for 5 consecutive days. As no alterations or disturbs were observed, treatment was carried out on subjects suffering from seborrheic dermatitis with dandruff.

The treatment was effected on 7 subjects, with different severity of the condition, from dry dandruff to yellow-red scaling papules.

Treatment was carried out distributing some shampoo on the scalp, massaging it and leaving it on for some minutes, then thoroughly rinsing the scalp with water and repeating the operation.

This treatment was carried out every other day until the dandruff was controlled, then twice a week.

Marked reduction or even disappearance of dandruff was observed, thereby proving the effectiveness of calcium trifluoroacetate in the treatment of seborrheic dermatitis and dandruff.

## Claims

1. The use of calcium trifluoroacetate for the preparation of a topical medicament for the treatment of psoriasis, erythematous dermatitis, seborrheic dermatitis, dandruff

## Patentansprüche

1. Verwendung von Calciumtrifluoracetat zur Herstellung eines topischen Arzneimittels zur Behandlung von Psoriasis, erythematöse Dermatitis, serborrhoische Dermatitis, Schuppen.

## Revendications

1. Utilisation du trifluoroacétate de calcium pour la préparation d'un médicament topique pour le traitement du psoriasis, de la dermatite érythémateuse, de la dermatite séborrhéique, des pellicules.
